# EUROPÄISCHE PATENTSCHRIFT

(11) **EP 3 347 342 B1**
(45) Veröffentlichungstag und Bekanntmachung des Hinweises auf die Patenterteilung: **24.04.2019**
(21) Anmeldenummer: 16760497.4
(22) Anmeldetag: 05.09.2016
(51) Int. Cl.: C07C 277/08, C07C 279/26

(54) **VERFAHREN ZUR HERSTELLUNG VON BIGUANIDIN-SALZEN UND S-TRIAZINEN**
METHOD FOR THE PREPARATION OF BIGUANIDINE SALTS AND S-TRIAZINES
PROCEDE DE FABRICATION DE SELS DE BIGUANIDINE ET TRIAZINES S

(30) Priorität: 11.09.2015 EP 15184873
(43) Veröffentlichungstag der Anmeldung: 18.07.2018
(73) Patentinhaber: Bayer CropScience Aktiengesellschaft, 40789 Monheim am Rhein (DE)
(72) Erfinder: RESSEL, Hans-Joachim, 65795 Hattersheim (DE); FORD, Mark James, 65207 Wiesbaden-Breckenheim (DE)
(74) Vertreter: BIP Patents
(86) Internationale Anmeldenummer: PCT/EP2016/070858
(87) Internationale Veröffentlichungsnummer: WO 2017/042126

(56) Entgegenhaltungen:
- WO-A1-2008/026757
- WO-A1-2009/077059

## Beschreibung

Die Erfindung betrifft ein Verfahren zur Herstellung von Biguanidin-Salzen und s-Triazinen.

Symmetrische Triazine, d.h. 1,3,5-Triazine oder s-Triazine genannt, sind je nach Art der Substituenten wichtige Zwischenprodukte oder gar selbst Bestandteil vieler pharmazeutischer und agrochemischer Wirkstoffe. Ihre Herstellung erfolgt häufig über Biguanidine, die dann beispielsweise mit einem Carbonsäure-Derivat zu den gewünschten s-Triazinen reagieren. Diese Reaktionen verlaufen oft mit nur geringen Ausbeuten, sodass zur Steigerung der Ausbeute große Überschüsse der meist teuren Carbonsäure-Derivate verwendet werden. Ein weiterer Nachteil der bekannten Methoden besteht darin, dass bei der Reaktion eines Biguanidins mit einem chiralen Carbonsäure-Derivat, wie Milchsäureester oder α-Halogen- Carbonsäureester, die Reaktion unter grossem Verlust der stereochemischen Information am α-Kohlenstoffatom des Carbonsäure-Derivats verläuft.

Bekannte Methoden zur Herstellung von Biguanidin-Salzen sind die Reaktion von Cyanamid mit Guanidinen und die Reaktion von Cyanoguanidin mit Ammoniumsalzen bei hohen Temperaturen in Lösung oder in Schmelze. Diese Methoden weisen jedoch die Nachteile auf, dass sie zu unzureichenden Ausbeuten und unzureichenden Reinheiten führen, oder auch zu Mischungen, aus denen das gewünschte Biguanidin-Salz schwer zu isolieren ist. So kann das gebildete Biguanidin-Salz thermisch in Guanidin-Derivate zerfallen, die die weitere Verwendung stören. Auch unter Sicherheitsaspekten sind diese Methoden als nachteilig anzusehen. So verlaufen Zersetzungsreaktionen häufig stark exotherm schon bei niedriger Onset-Temperatur. Ein weiteres Sicherheitsproblem stellt die Akkumulation von Cyanoguanidin im Reaktionsverlauf bei der Umsetzung mit Ammoniumsalzen dar.

Zur Überwindung dieser Nachteile wird in WO 2009/077059 A1 vorgeschlagen, Amine, beziehungsweise deren Hydrochloride, mit Cyanoguanidin mit Aluminiumalkoholaten zu intermediären Biguanidino-Aluminium-Komplexen umzusetzen, die dann mit Carbonsäure-Derivaten zu s-Triazinen weiter reagieren. Zwar verläuft diese Reaktion mit sehr guten Ausbeuten, jedoch führen die benötigten großen Überschüsse von Cyanoguanidin sowie von Aluminium-Alkoholaten zu erheblichen Abfallmengen, die aufwändig entsorgt werden müssen.

Aufgabe vorliegender Erfindung war die Bereitstellung eines Verfahrens zur Herstellung von Biguanidin-Salzen und s-Triazinen, das die Nachteile der aus dem Stand der Technik bekannten Verfahren überwindet.

Es wurde nun ein Verfahren zur Herstellung von Biguanidin-Salzen und s-Triazinen gefunden, das
- mit einer Vielzahl strukturell unterschiedlicher Amine durchgeführt werden kann,
- nur einen geringen Überschuss an Cyanoguanidin benötigt,
- ohne Akkumulation von Cyanoguanidin verläuft,
- zu lediglich geringen Mengen an Nebenprodukten führt,
- zu hohen Ausbeuten führt, und
- ohne Verlust von stereochemischen Informationion verläuft.

Wesentliche Merkmale des erfindungsgemäßen Verfahrens liegen in der Verwendung bestimmter Lösungsmittel und darin begründet, dass das herzustellende Biguanidin-Salz zu Beginn der Reaktion in katalytischen Mengen zugegeben wird. Es wurde festgestellt, dass das Biguanidin-Salz autokatalytisch reagiert und die oben genannten Vorteile insbesondere dann gegeben sind, wenn katalytische Mengen des herzustellenden Biguanidin-Salzes zu Beginn der Reaktion zugegeben werden. Solche katalytischen Mengen können durch Vorversuche, gegebenenfalls auch durch andere Verfahren (jeweils mit geringeren Ausbeuten), gewonnen werden und dann im erfindungsgemäßen Verfahren eingesetzt werden. Das so hergestellte Biguanidin-Salz kann
A) isoliert und als Intermediat gelagert werden, oder
B) direkt mit einem geeigneten Reagens zu einem 1,3,5-Triazin umgesetzt werden.

Ein Gegenstand vorliegender Erfindung ist somit A) ein Verfahren zur Herstellung von Biguanidin-Salzen der Formel (III), das dadurch gezeichnet ist, dass
a) ein Amin der Formel (I) mit einer Säure H⁺A⁻ in ein Salz (II) überführt wird,
b) unter Rühren bei erhöhter Temperatur Cyanoguanidin und 0,5 bis 10 Molprozent des herzustellenden Biguanidin-Salzes (III), bezogen auf das Amin der Formel (I), in einem polaren aprotischen Lösungsmittel zudosiert wird,
c) das so erhaltene Reaktionsgemisch nach Abkühlung abgesaugt, mit Lösungsmittel gewaschen und getrocknet wird, und
d) worin die Substituenten wie nachfolgend definiert sind: 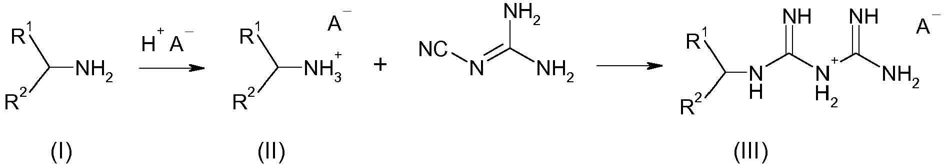
   R¹ bedeutet Wasserstoff, (C₁-C₈)-Alkyl, (C₃-C₈)-Cycloalkyl, Phenyl, Naphthyl, (C₁-C₄)-Alkylphenyl, wobei die vier letztgenannten Reste durch n Reste aus der Gruppe bestehend aus Methyl, Ethly, Propyl und Methoxy substituiert sind,
   R² bedeutet (C₁-C₈)-Alkyl, (C₃-C₈)-Cycloalkyl, Phenyl, Naphthyl, Phenyl-(C₁-C₄)-Alkyl, wobei diese vier vorstehend genannten Reste durch n Reste aus der Gruppe bestehend aus Methyl, Ethly, Propyl, Methoxy und (C₃-C₄)-Cycloalkyl substituiert sind, oder
   R¹ und R² bilden gemeinsam mit dem Kohlenstoffatom, an dem sie gebunden sind, einen Rest aus der Gruppe bestehend aus (C₃-C₈)-Cycloalkyl, Phenyl-(C₃-C₈)-Cycloalkyl, 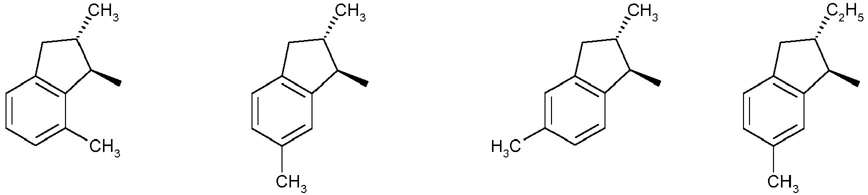 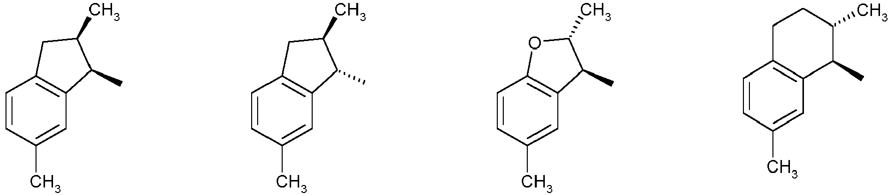 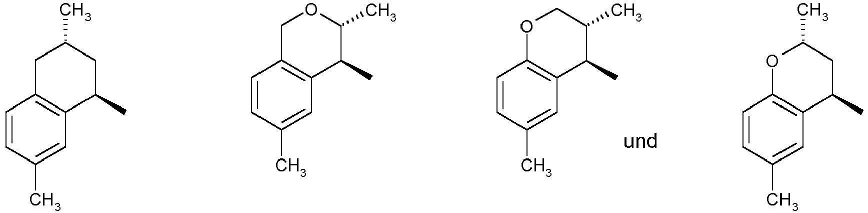
   A⁻ bedeutet ein Anion einer Säure,
   n bedeutet 0, 1, 2 oder 3.

Ein weiterer Gegenstand vorliegender Erfindung ist B) ein Verfahren zur Herstellung von s-Triazinen der Formel (V), das dadurch gezeichnet ist, dass
a) ein Amin der Formel (I) mit einer Säure H⁺A⁻ in ein Salz (II) überführt wird,
b) unter Rühren bei erhöhter Temperatur Cyanoguanidin und 0,5 bis 10 Molprozent des herzustellenden Biguanidin-Salzes (III), bezogen auf das Amin der Formel (I), in einem polaren aprotischen Lösungsmittel zudosiert wird,
c) nach vollständiger Umsetzung ein Phasentransfer-Katalysator und eine Base zugegeben werden,
d) ein Carbonsäure-Derivat der Formel (IV) zudosiert wird, und
e) worin die Substituenten wie nachfolgend definiert sind:

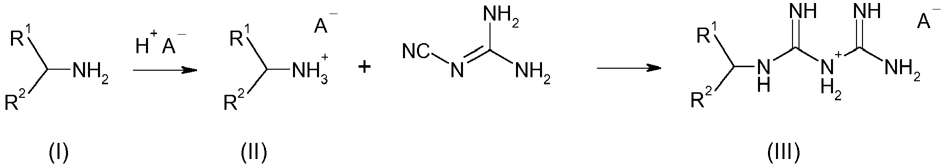

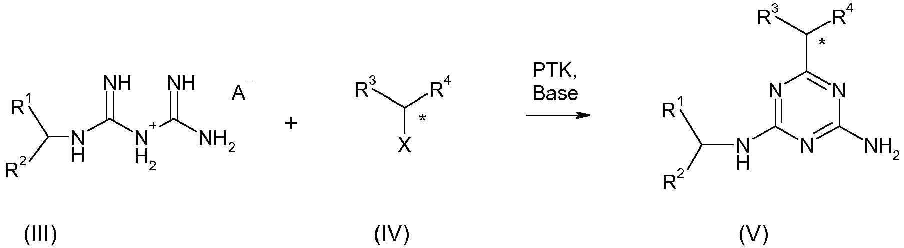

R¹ bedeutet Wasserstoff, (C₁-C₈)-Alkyl, (C₃-C₈)-Cycloalkyl, Phenyl, Naphthyl, (C₁-C₄)-Alkylphenyl, wobei die vier letztgenannten Reste durch n Reste aus der Gruppe bestehend aus Methyl, Ethly, Propyl und Methoxy substituiert sind,
R² bedeutet (C₁-C₈)-Alkyl, (C₃-C₈)-Cycloalkyl, Phenyl, Naphthyl, Phenyl-(C₁-C₄)-Alkyl, wobei diese vier vorstehend genannten Reste durch n Reste aus der Gruppe bestehend aus Methyl, Ethly, Propyl, Methoxy und (C₃-C₄)-Cycloalkyl substituiert sind, oder
R¹ und R² bilden gemeinsam mit dem Kohlenstoffatom, an dem sie gebunden sind, einen Rest aus der Gruppe bestehend aus (C₃-C₈)-Cycloalkyl, Phenyl-(C₃-C₈)-Cycloalkyl, 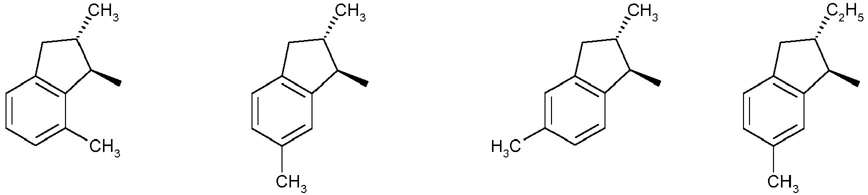 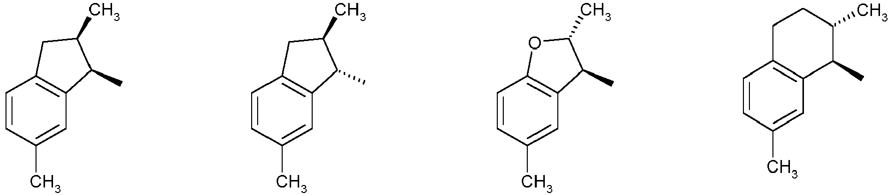 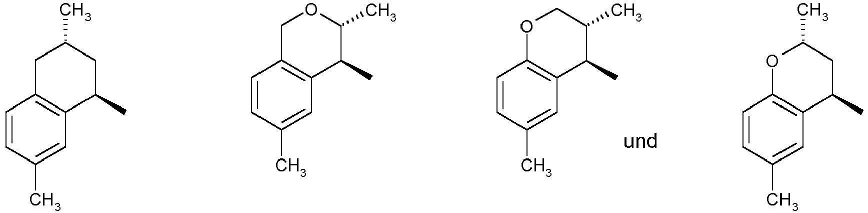
A⁻ bedeutet ein Anion einer Säure,
n bedeutet 0, 1, 2 oder 3,
R³ bedeutet Wasserstoff, Halogen, (C₁-C₆)-Alkyl, (C₁-C₆)-Alkoxy, (C₃-C₈)-Cycloalkyl, Phenyl, wobei die drei letztgenannten Reste durch n Reste aus der Gruppe bestehend aus Methyl, Ethly, Propyl und Methoxy substituiert sind,
R⁴ bedeutet Wasserstoff, Halogen, (C₁-C₆)-Alkyl, (C₁-C₆)-Alkoxy, (C₃-C₈)-Cycloalkyl, Phenyl, wobei die drei letztgenannten Reste durch n Reste aus der Gruppe bestehend aus Methyl, Ethly, Propyl und Methoxy substituiert sind,
X bedeutet (C₁-C₆)-Alkoxycarbonyl, Cyano oder Chlorcarbonyl.

Das mit einem Stern (*) gekennzeichnete Kohlenstoffatom bedeutet ein Chiralitätszentrum, sofern R³ und R⁴ unterschiedlich sind und keiner dieser Reste Wasserstoff bedeutet.

In den Formel (I), (II), (III), (IV), (V) und nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl.

Halogen bedeutet Fluor, Chlor Brom oder Iod, bevorzugt Fluor oder Chlor.

Ist eine Gruppe mehrfach durch Reste substituiert, so ist darunter zu verstehen, daß diese Gruppe durch ein oder mehrere gleiche oder verschiedene der genannten Reste substituiert ist.

Das Anion A⁻ bedeutet beispielsweise Cl⁻, Br⁻, I⁻, HSO₄⁻, HCO₃⁻ oder H₃CSO₃⁻.

In einer bevorzugten Ausführungsform des Erfindungsgegenstands A) haben die Substituenten folgende Bedeutung:
R¹ bedeutet Wasserstoff, (C₁-C₈)-Alkyl, (C₃-C₈)-Cycloalkyl, Phenyl, (C₁-C₄)-Alkylphenyl, wobei die drei letztgenannten Reste durch n Reste aus der Gruppe bestehend aus Methyl, Ethly, Propyl und Methoxy substituiert sind,
R² bedeutet (C₁-C₈)-Alkyl, (C₃-C₈)-Cycloalkyl, Phenyl, Naphthyl oder Phenyl-(C₁-C₄)-Alkyl,
   oder
R¹ und R² bilden gemeinsam mit dem Kohlenstoffatom, an dem sie gebunden sind, einen Rest aus der Gruppe bestehend aus (C₃-C₈)-Cycloalkyl, Phenyl-(C₃-C₈)-Cycloalkyl, 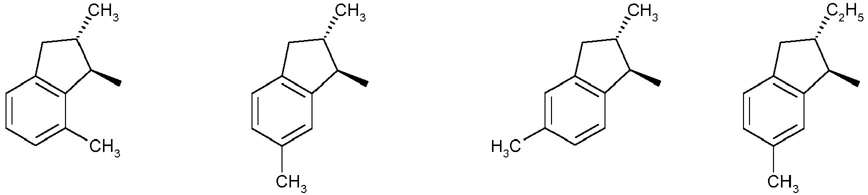 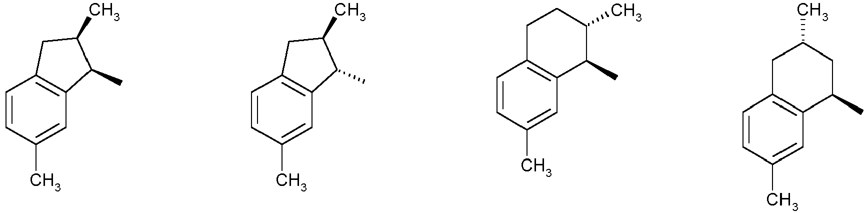
   A⁻ bedeutet Cl⁻, Br⁻ oder HSO₄⁻,
   n bedeutet 0, 1, 2 oder 3.

In einer bevorzugten Ausführungsform des Erfindungsgegenstands B) haben die Substituenten folgende Bedeutung:
R¹ bedeutet Wasserstoff, (C₁-C₈)-Alkyl, (C₃-C₈)-Cycloalkyl, Phenyl, (C₁-C₄)-Alkylphenyl, wobei die drei letztgenannten Reste durch n Reste aus der Gruppe bestehend aus Methyl, Ethly, Propyl und Methoxy substituiert sind,
R² bedeutet (C₁-C₈)-Alkyl, (C₃-C₈)-Cycloalkyl, Phenyl, Phenyl-(C₁-C₄)-Alkyl, oder
R¹ und R² bilden gemeinsam mit dem Kohlenstoffatom, an dem sie gebunden sind, einen Rest aus der Gruppe bestehend aus (C₃-C₈)-Cycloalkyl, Phenyl-(C₃-C₈)-Cycloalkyl, 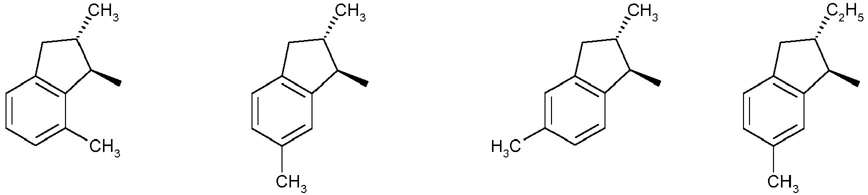 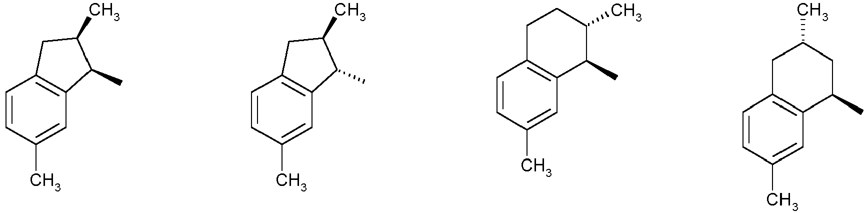
   - A⁻: bedeutet Cl⁻, Br⁻ oder HSO₄⁻,
   - n: bedeutet 0, 1, 2 oder 3,
   - R³: bedeutet Wasserstoff, Chlor, Fluor, (C₁-C₆)-Alkyl oder (C₁-C₆)-Alkoxy,
   - R⁴: bedeutet Wasserstoff, Chlor, Fluor, (C₁-C₆)-Alkyl oder (C₁-C₆)-Alkoxy,
   - X: bedeutet (C₁-C₆)-Alkoxycarbonyl.

In einer besonders bevorzugten Ausführungsform des Erfindungsgegenstands A) haben die Substituenten folgende Bedeutung:
R¹ und R² bilden gemeinsam mit dem Kohlenstoffatom, an dem sie gebunden sind, den Rest 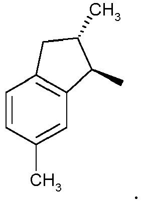
A⁻ bedeutet Cl⁻.

In einer besonders bevorzugten Ausführungsform des Erfindungsgegenstands B) haben die Substituenten folgende Bedeutung:
R¹ und R² bilden gemeinsam mit dem Kohlenstoffatom, an dem sie gebunden sind, den Rest 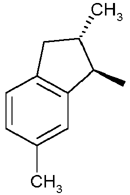
A⁻ bedeutet Cl⁻,
R³ bedeutet Fluor,
R⁴ bedeutet Methyl,
X bedeutet (C₁-C₆)-Alkoxycarbonyl.

Die Amin-Salze (II) können durch Umsetzung der freien Amine der Formel (I) mit einer Säure, wie Chlorwasserstoff, Bromwasserstoff, lodwasserstoff, Schwefelsäure, Salpetersäure, Kohlensäure, Sulfonsäuren wie Methansulfonsäure, gemäß dem Fachmann bekannten Methoden hergestellt werden. Die freien Amine sind kommerziell erhältlich, oder können nach einfachen dem Fachmann bekannten Methoden hergestellt werden.

Die Reaktion in Schritt b) beider oben genannten Erfindungsgegenstände A) und B) wird üblicherweise bei einer Temperatur von 100°C bis zum Siedepunkt des Lösungsmittel, vorzugsweise von 140°C bis 148°C, und mit einem Überschuss von 5 bis 10% an Cyanoguanidin, bezogen auf das Amin-Salz der Formel (II), durchgeführt. Geeignete Lösungsmittel sind Anisol, n-Hexylacetat, Dichlorbenzole und deren Mischungen. Bevorzugt ist Anisol. Diese Reaktion kann bei Atmosphärendruck und bei erhöhtem Druck durchgeführt werden.

Die Reaktion in Schritt b) beider oben genannten Erfindungsgegenstände A) und B) wird vorzugsweise mit 0,5 bis 2 Molprozent des herzustellenden Biguanidin-Salzes (III) durchgeführt.

Als Phasentransfer-Katalysatoren in Schritt c) des zweitgenannten Erfindungsgegenstands B) können eine Vielzahl dem Fachmann bekannte verwendet werden. Geeignete Phasentransfer-Katalysatoren sind Polyethylenglycole, quaternäre Ammoniumsalze und Kronenether. Bevorzugt sind Polyethylenglycole wie PEG 2000.

Der Phasentransfer-Katalysator in Schritt c) des zweitgenannten Erfindungsgegenstands B) wird üblicherweise in einer Menge von 0 bis 10, bevorzugt 0,5 bis 5, besonders bevorzugt 0,5 bis 3, ganz besonders bevorzugt 0,5 bis 1,5 Molprozent, bezogen auf das Amin der Formel (I), zugegeben.

Die Base in Schritt c) des zweitgenannten Erfindungsgegenstands B) wird üblicherweise in einer Menge von 100 bis 500, bevorzugt 100-400, besonders bevorzugt 200 bis 400 Molprozent, bezogen auf das Amin der Formel (I), zugegeben.

Das Carbonsäure-Derivat der Formel (IV) in Schritt c) des zweitgenannten Erfindungsgegenstands B) wird zweckmäßigerweise in einer Menge von 100 bis 250, bevorzugt 100-150, besonders bevorzugt 100-140 Molprozent, bezogen auf das Amin der Formel (I), eingesetzt.

Als Basen in Schritt c) und d) des zweitgenannten Erfindungsgegenstands B) sind die Carbonate der Alkali- und der Erdalkalimetalle geeignet. Besonders geeignet sind Kaliumcarbonat, Natriumcarbonat, Lithiumcarbonat, Calciumcarbonat und Magnesiumcarbonat. Bevorzugt sind Kaliumcarbonat und Natriumcarbonat, besonders bevorzugt ist Kaliumcarbonat.

Die einzelnen Schritte des erfindungsgemäßen Verfahrens werden zweckmäßigerweise unter Schutzgas durchgeführt.

Die nachfolgenden Ausführungsbeispiele erläutern die Erfindung näher.

### Beispiel 1 (gemäß Erfindungsgegenstand A)

Herstellung von Amino-N{N-[(1*R*,2*S*)-2,6-dimethyl-2,3-dihydro-1H-inden-1-yl]carbamimidoyl} iminomethanaminiumchlorid:
13,03 g (0,080mol) (1*R*,2*S*)-2,6-dimethyl-2,3-dihydro-1H-inden-1-yl-amin wurden unter Argonatmospäre in 110 ml Methoxybenzol vorgelegt und auf 0°C abgekühlt. Dann wurden 8,26 g (0,082mol) conc. Salzsäure (36%-ig) unter Rühren zugetropft. Man ließ bis auf Raumtemperatur kommen und rührte für 30 min nach. Dann wurde im Vakuum (20 mbar) auf 100-120°C im Bad erhitzt und ein Gemisch aus Wasser und Methoxybenzol abdestilliert bis kein Wasser mehr überging. Es wurden dann weiter unter Argonatmospäre 1,22 g (4,5 mmol) des herzustellenden Amino-N{N-[(1*R*,2*S*)-2,6-dimethyl-2,3-dihydro-1H-inden-1-yl]carbamimidoyl} iminomethanaminiumchlorids (erhalten aus einem Vorversuch) zugefügt und anschließend über eine Feststoff-Dosierung bei 145-149°C innerhalb von 2,5 Stunden 7,5 g (88 mmol) Cyanoguanidin zugegeben. Das Fortschreiten der Reaktion wurde mittels HPLC kontrolliert. Man ließ noch für 1,5 Stunden bei 145-148°C nachrühren und danach auf 75°C abkühlen. Dann wurden 8 ml Methanol zugegeben und unter Rühren weiter abgekühlt bis 20°C. Das kristalline Produkt wurde im Vakuum über eine Fritte abgesaugt und 2-mal mit je 10 ml Ethylacetat gewaschen. Nach dem Trocknen im Vakuum (70°C / 15 mbar) erhielt man 22,25 g Amino-N{N-[(1*R*,2*S*)-2,6-dimethyl-2,3-dihydro-1H-inden-1-yl]carbamimidoyl} iminomethan-aminiumchlorid mit einer Reinheit von 99%. Dies entspricht unter Berücksichtigung der katalytisch eingesetzten Menge einer Ausbeute von 92,4% der Theorie.

### Beispiel 2 (gemäß Erfindungsgegenstand A)

Analog zu den in Beispiel 1 genannten Bedingungen erhielt man aus 1-Cyclobutyl-3-phenylpropylamin das entsprechende Biguanidinchlorid in einer Ausbeute von 78%.

### Beispiel 3 (gemäß Erfindungsgegenstand A)

Analog zu den in Beispiel 1 genannten Bedingungen erhielt man aus (1*R*,2*S*)-2,6-dimethyl-2,3-dihydro-1H-inden-1-yl-amin und Kohlensäure das entsprechende Biguanidincarbonat in einer Ausbeute von 90%.

### Beispiel 4 (gemäß Erfindungsgegenstand A)

Analog zu den in Beispiel 1 genannten Bedingungen erhielt man aus (1*R*,2*S*)-2,6-dimethyl-2,3-dihydro-1H-inden-1-yl-amin und Bromwasserstoff das entsprechende Biguanidinbromid in einer Ausbeute von 69%.

### Beispiel 5 (gemäß Erfindungsgegenstand B)

Herstellung von N-[(1*R*,2*S*)-2,6-Dimethyl-2,3-dihydro-1H-inden-1yl]-6-[(1*R*)-1-fluorethyl]-1,3,5-triazin-2,4-diamin:
12,0 g (73,6 mmol) (1*R*,2*S*)-2,6-dimethyl-2,3-dihydro-1H-inden-1-yl-amin wurden unter Stickstoffatmospäre in 170 ml Methoxybenzol vorgelegt und auf 5°C abgekühlt. Dann wurden 7,61 g (75,1mmol) conc. Salzsäure (36%-ig) zudosiert und 30 Minuten gerührt. Dann wurde bei 80 mbar unter Erwärmung auf 90-115°C ein Gemisch aus Wasser und Methoxybenzol abdestilliert bis kein Wasser mehr überging.

Zur verbleibenden Suspension wurden dann 0,52 g (1,84 mmol) des herzustellenden Amino-N{N-[(1R,2S)-2,6-dimethyl-2,3-dihydro-1H-inden-1-yl]carbamimidoyl}-iminomethanaminiumchlorids (erhalten aus einem Vorversuch) zugefügt und auf 145°C erhitzt. Bei 145-147°C wurden dann innerhalb von 2 Stunden 6,88 g (81,0mmol) Cyanoguanidin zugegeben. Es wurde noch 4 Stunden bei ca. 147°C nachgerührt und dann abgekühlt.

Bei 90°C wurden zunächst 0,5 g PEG 2000 und 41,1g (295 mmol) trockenes, pulverisiertes Kaliumcarbonat zugegeben. Bei 95°C wurden anfangs 9,0 g und nach 1 Stunde weitere 2,0 g (insgesamt 103 mmol) (R)-Methyl-2-fluorpropionat zudosiert. Das Reaktionsgemisch wurde für insgesamt 5 Stunden bei 95°C gerührt.

Dann wurde auf 50°C abgekühlt und abfiltriert, dann 2-mal mit je 10 ml Methoxybenzol warm gewaschen. Die Filtrate wurden am Rotationsverdampfer eingedampft. Der Rückstand wurde in 40 ml Methanol aufgenommen und das Produkt nach und nach durch Zugabe von Wasser gefällt. Der Feststoff wurde abgesaugt, 2-mal mit Wasser gewaschen und bis zur Gewichtskonstanz getrocknet im Vakuumtrockenschrank. Man erhielt 20,5 g N-[(1*R*,2*S*)-2,6-Dimethyl-2,3-dihydro-1H-inden-1yl]-6-[(1*R*)-1-fluorethyl]-1,3,5-triazin-2,4-diamin.

### Beispiel 6 (gemäß Erfindungsgegenstand B)

Analog zu den in Beispiel 5 genannten Bedingungen erhielt man aus Benzylamin das entsprechende Triazin in einer Ausbeute von 58%.

### Beispiel 7 (gemäß Erfindungsgegenstand B)

Analog zu den in Beispiel 5 genannten Bedingungen erhielt man aus cis-4-Phenyl-cyclohexylamin das entsprechende Triazin in einer Ausbeute von 51%.

## Patentansprüche

1. Verfahren zur Herstellung von Biguanidin-Salzen der Formel (III), das dadurch gezeichnet ist, dass
a) ein Amin der Formel (I) mit einer Säure H⁺A⁻ in ein Salz (II) überführt wird,
b) unter Rühren bei erhöhter Temperatur Cyanoguanidin und 0,5 bis 10 Molprozent des herzustellenden Biguanidin-Salzes (III), bezogen auf das Amin der Formel (I), in einem polaren aprotischen Lösungsmittel zudosiert wird,
c) das so erhaltene Reaktionsgemisch nach Abkühlung abgesaugt, mit Lösungsmittel gewaschen und getrocknet wird, und
d) worin die Substituenten wie nachfolgend definiert sind: 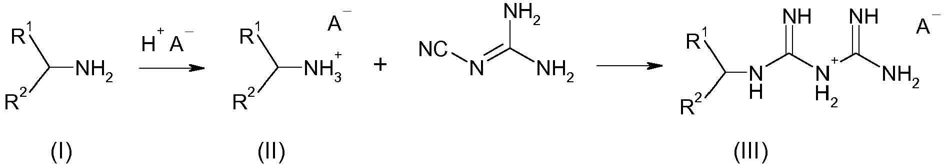
R¹ bedeutet Wasserstoff, (C₁-C₈)-Alkyl, (C₃-C₈)-Cycloalkyl, Phenyl, Naphthyl, (C₁-C₄)-Alkylphenyl, wobei die vier letztgenannten Reste durch n Reste aus der Gruppe bestehend aus Methyl, Ethly, Propyl und Methoxy substituiert sind,
R² bedeutet (C₁-C₈)-Alkyl, (C₃-C₈)-Cycloalkyl, Phenyl, Naphthyl, Phenyl-(C₁-C₄)-Alkyl, wobei diese vier vorstehend genannten Reste durch n Reste aus der Gruppe bestehend aus Methyl, Ethly, Propyl, Methoxy und (C₃-C₄)-Cycloalkyl substituiert sind, oder
R¹ und R² bilden gemeinsam mit dem Kohlenstoffatom, an dem sie gebunden sind, einen Rest aus der Gruppe bestehend aus (C₃-C₈)-Cycloalkyl, Phenyl-(C₃-C₈)-Cycloalkyl, 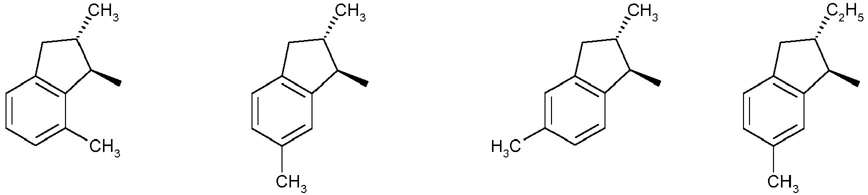 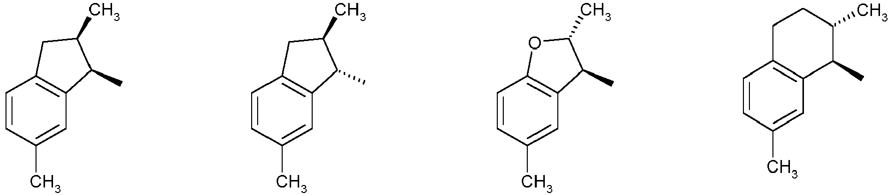 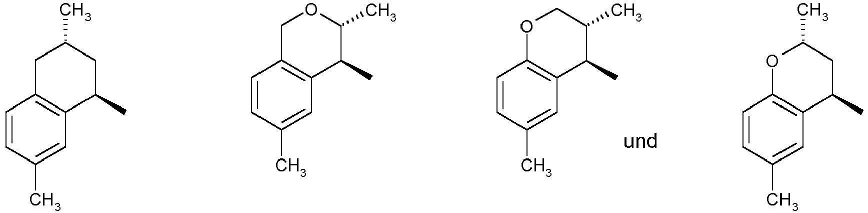
A⁻ bedeutet ein Anion einer Säure,
n bedeutet 0, 1, 2 oder 3.

2. Verfahren nach Anspruch 1, worin
R¹ bedeutet Wasserstoff, (C₁-C₈)-Alkyl, (C₃-C₈)-Cycloalkyl, Phenyl, (C₁-C₄)-Alkylphenyl, wobei die drei letztgenannten Reste durch n Reste aus der Gruppe bestehend aus Methyl, Ethly, Propyl und Methoxy substituiert sind,
R² bedeutet (C₁-C₈)-Alkyl, (C₃-C₈)-Cycloalkyl, Phenyl, Naphthyl oder Phenyl-(C₁-C₄)-Alkyl,
oder
R¹ und R² bilden gemeinsam mit dem Kohlenstoffatom, an dem sie gebunden sind, einen Rest aus der Gruppe bestehend aus (C₃-C₈)-Cycloalkyl, Phenyl-(C₃-C₈)-Cycloalkyl, 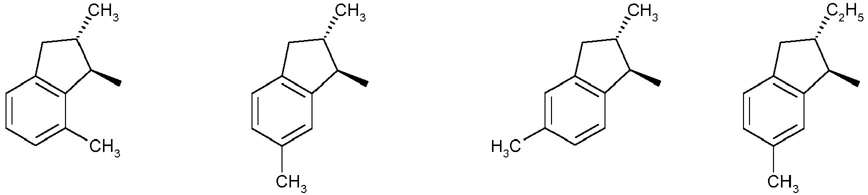 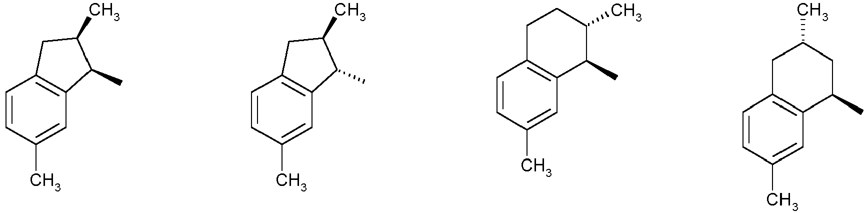 A⁻ bedeutet Cl⁻, Br⁻ oder HSO₄⁻,
n bedeutet 0, 1, 2 oder 3.

3. Verfahren nach Anspruch 1 oder 2, worin
R¹ und R² gemeinsam mit dem Kohlenstoffatom, an dem sie gebunden sind, den Rest 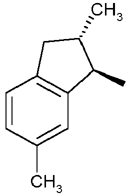 bilden,
und
A⁻ bedeutet Cl⁻.

4. Verfahren zur Herstellung von s-Triazinen der Formel (V), das dadurch gezeichnet ist, dass
a) ein Amin der Formel (I) mit einer Säure H⁺A⁻ in ein Salz (II) überführt wird,
b) unter Rühren bei erhöhter Temperatur Cyanoguanidin und 0,5 bis 10 Molprozent des herzustellenden Biguanidin-Salzes (III), bezogen auf das Amin der Formel (I), in einem polaren aprotischen Lösungsmittel zudosiert wird,
c) nach vollständiger Umsetzung ein Phasentransfer-Katalysator und eine Base zugegeben werden,
d) ein Carbonsäure-Derivat der Formel (IV) zudosiert wird, und
e) worin die Substituenten wie nachfolgend definiert sind: 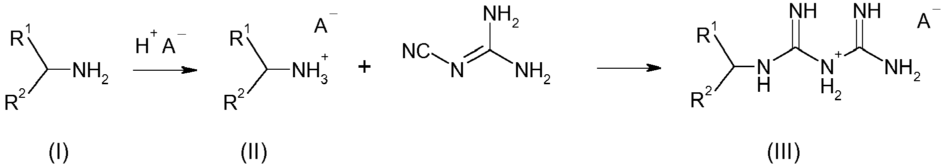 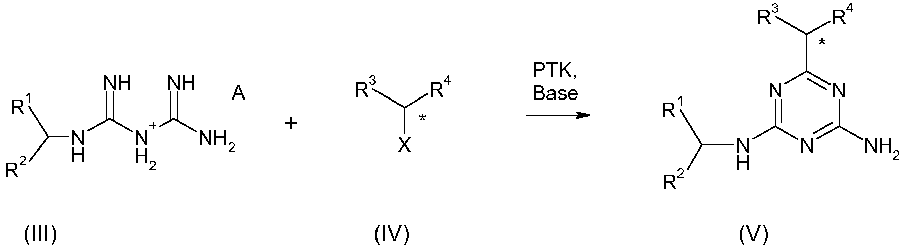
R¹ bedeutet Wasserstoff, (C₁-C₈)-Alkyl, (C₃-C₈)-Cycloalkyl, Phenyl, Naphthyl, (C₁-C₄)-Alkylphenyl, wobei die vier letztgenannten Reste durch n Reste aus der Gruppe bestehend aus Methyl, Ethly, Propyl und Methoxy substituiert sind,
R² bedeutet (C₁-C₈)-Alkyl, (C₃-C₈)-Cycloalkyl, Phenyl, Naphthyl, Phenyl-(C₁-C₄)-Alkyl, wobei diese vier vorstehend genannten Reste durch n Reste aus der Gruppe bestehend aus Methyl, Ethly, Propyl, Methoxy und (C₃-C₄)-Cycloalkyl substituiert sind, oder
R¹ und R² bilden gemeinsam mit dem Kohlenstoffatom, an dem sie gebunden sind, einen Rest aus der Gruppe bestehend aus (C₃-C₈)-Cycloalkyl, Phenyl-(C₃-C₈)-Cycloalkyl, 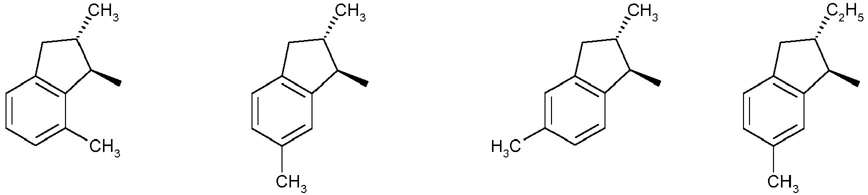 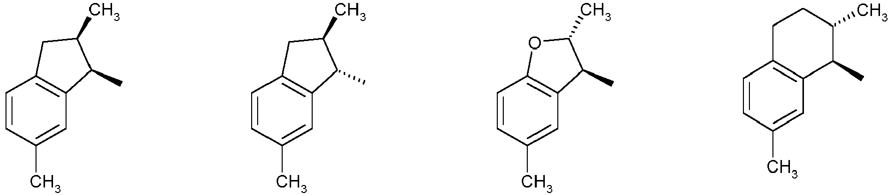 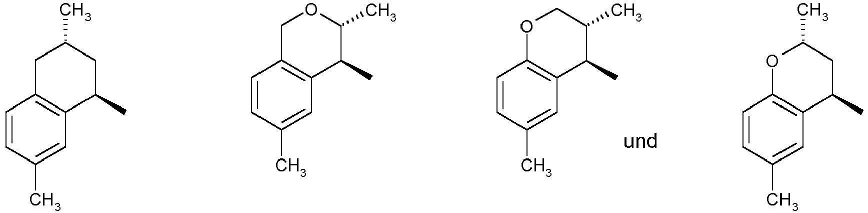
A⁻ bedeutet ein Anion einer Säure,
n bedeutet 0, 1, 2 oder 3,
R³ bedeutet Wasserstoff, Halogen, (C₁-C₆)-Alkyl, (C₁-C₆)-Alkoxy, (C₃-C₈)-Cycloalkyl, Phenyl, wobei die drei letztgenannten Reste durch n Reste aus der Gruppe bestehend aus Methyl, Ethly, Propyl und Methoxy substituiert sind,
R⁴ bedeutet Wasserstoff, Halogen, (C₁-C₆)-Alkyl, (C₁-C₆)-Alkoxy, (C₃-C₈)-Cycloalkyl, Phenyl, wobei die drei letztgenannten Reste durch n Reste aus der Gruppe bestehend aus Methyl, Ethly, Propyl und Methoxy substituiert sind,
X bedeutet (C₁-C₆)-Alkoxycarbonyl, Cyano oder Chlorcarbonyl.

5. Verfahren nach Anspruch 4, worin
R¹ bedeutet Wasserstoff, (C₁-C₈)-Alkyl, (C₃-C₈)-Cycloalkyl, Phenyl, (C₁-C₄)-Alkylphenyl, wobei die drei letztgenannten Reste durch n Reste aus der Gruppe bestehend aus Methyl, Ethly, Propyl und Methoxy substituiert sind,
R² bedeutet (C₁-C₈)-Alkyl, (C₃-C₈)-Cycloalkyl, Phenyl, Phenyl-(C₁-C₄)-Alkyl, oder
R¹ und R² bilden gemeinsam mit dem Kohlenstoffatom, an dem sie gebunden sind, einen Rest aus der Gruppe bestehend aus (C₃-C₈)-Cycloalkyl, Phenyl-(C₃-C₈)-Cycloalkyl, 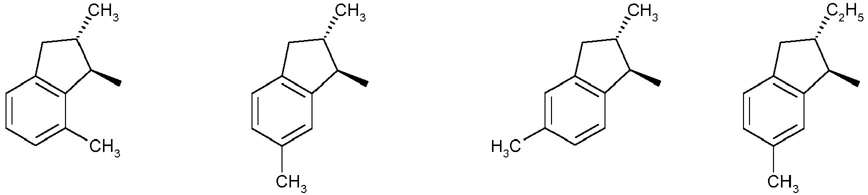 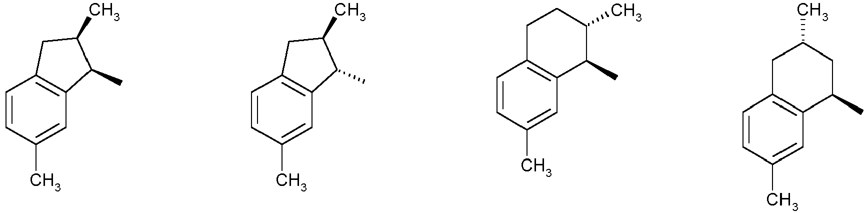 A⁻ bedeutet Cl⁻, Br⁻ oder HSO₄⁻,
n bedeutet 0, 1, 2 oder 3,
R³ bedeutet Wasserstoff, Chlor, Fluor, (C₁-C₆)-Alkyl oder (C₁-C₆)-Alkoxy,
R⁴ bedeutet Wasserstoff, Chlor, Fluor, (C₁-C₆)-Alkyl oder (C₁-C₆)-Alkoxy,
X bedeutet (C₁-C₆)-Alkoxycarbonyl.

6. Verfahren nach Anspruch 4 oder 5, worin
R¹ und R² bilden gemeinsam mit dem Kohlenstoffatom, an dem sie gebunden sind, den Rest 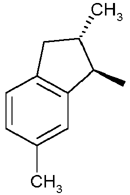 A⁻ bedeutet Cl⁻,
R³ bedeutet Fluor,
R⁴ bedeutet Methyl,
X bedeutet (C₁-C₆)-Alkoxycarbonyl.

7. Verfahren nach einem der Ansprüche 1 bis 6, wobei die Reaktion in Schritt b) bei einer Temperatur von 100°C bis zum Siedepunkt des Lösungsmittel und mit einem Überschuss von 5 bis 10% an Cyanoguanidin, bezogen auf das Amin-Salz der Formel (II), durchgeführt wird.

8. Verfahren nach Anspruch 7, wobei die Reaktion in Schritt b) in Anisol durchgeführt wird.

9. Verfahren nach einem der Ansprüche 1 bis 8, wobei die Reaktion in Schritt b) mit 0,5 bis 2 Molprozent des herzustellenden Biguanidin-Salzes (III) durchgeführt wird.

10. Verfahren nach einem der Ansprüche 4 bis 6, wobei die Reaktion in Schritt c) mit 0,5 bis 5 Molprozent, bezogen auf das Amin der Formel (I), eines Polyethylenglycols als Phasentransfer-Katalysator, durchgeführt wird.

11. Verfahren nach einem der Ansprüche 4 bis 6, wobei die Reaktion in Schritt c) mit 100-400 Molprozent, bezogen auf das Amin der Formel (I), einer Base aus der Gruppe bestehend aus Kaliumcarbonat, Natriumcarbonat, Lithiumcarbonat, Calciumcarbonat und Magnesiumcarbonat durchgeführt wird.

12. Verfahren nach Anspruch 11, wobei die Reaktion mit 100-150 Molprozent Kaliumcarbonat, bezogen auf das Amin der Formel (I), durchgeführt wird.

## Claims

1. A process for producing biguanidine salts of formula (III), wherein
a) an amine of formula (I) is converted into a salt (II) with an acid H⁺A⁻,
b) cyanoguanidine and 0.5 to 10 mol percent of the biguanidine salt (III) to be produced, based on the amine of formula (I), in a polar aprotic solvent are metered in with stirring at elevated temperature,
c) after cooling the thus obtained reaction mixture is suctioned off, washed with solvent and dried, and
d) where the substituents are as defined hereinbelow: 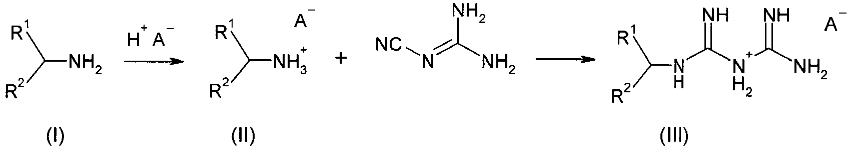
R¹ is hydrogen, (C₁-C₈)-alkyl, (C₃-C₈)-cycloalkyl, phenyl, naphthyl, (C₁-C₄)-alkylphenyl, wherein the four last-mentioned radicals are substituted by n radicals from the group consisting of methyl, ethyl, propyl and methoxy,
R² is (C₁-C₈)-alkyl, (C₃-C₈)-cycloalkyl, phenyl, naphthyl, phenyl-(C₁-C₄)-alkyl, wherein these four abovementioned radicals are substituted by n radicals from the group consisting of methyl, ethyl, propyl, methoxy and (C₃-C₄)-cycloalkyl,
or
R¹ and R² together with the carbon atom to which they are bonded form a radical from the group consisting of (C₃-C₈)-cycloalkyl, phenyl-(C₃-C₈)-cycloalkyl, 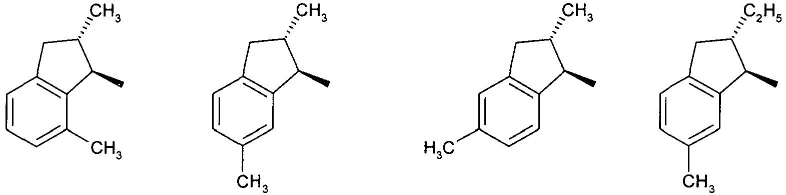 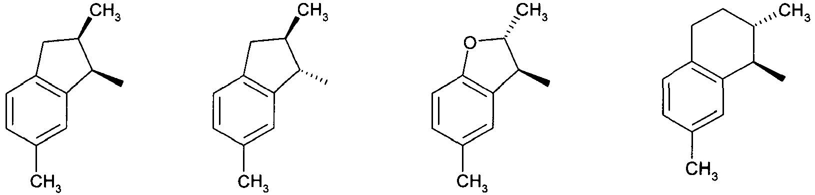 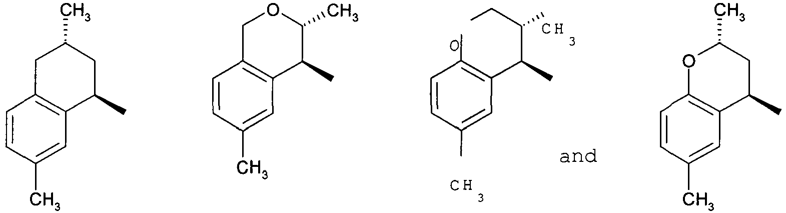
A⁻ is an anion of an acid,
n is 0, 1, 2 or 3.

2. Process according to Claim 1, wherein
R¹ is hydrogen, (C₁-C₈)-alkyl, (C₃-C₈)-cycloalkyl, phenyl, (C₁-C₄)-alkylphenyl, wherein the three last-mentioned radicals are substituted by n radicals from the group consisting of methyl, ethyl, propyl and methoxy,
R² is (C₁-C₈)-alkyl, (C₃-C₈)-cycloalkyl, phenyl, naphthyl or phenyl-(C₁-C₄)-alkyl,
or
R¹ and R² together with the carbon atom to which they are bonded form a radical from the group consisting of (C₃-C₈)-cycloalkyl, phenyl-(C₃-C₈)-cycloalkyl, 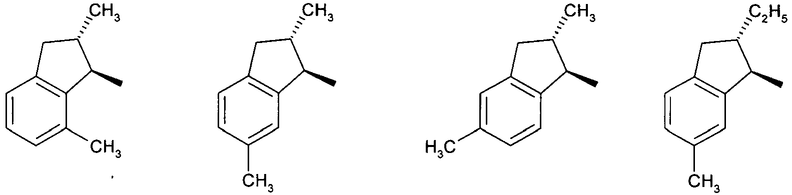 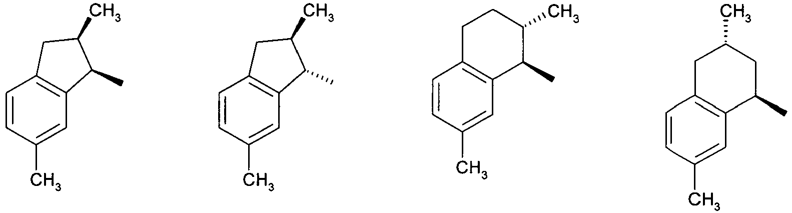 A⁻ is Cl⁻, Br⁻ or HSO₄⁻,
n is 0, 1, 2 or 3.

3. Process according to Claim 1 or 2, wherein
R¹ and R² together with the carbon atom to which they are bonded form the radical 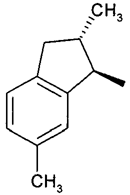 and
A⁻ is Cl⁻.

4. Process for producing s-triazines of formula (V), wherein
a) an amine of formula (I) is converted into a salt (II) with an acid H⁺A⁻,
b) cyanoguanidine and 0.5 to 10 mol percent of the biguanidine salt (III) to be produced, based on the amine of formula (I), in a polar aprotic solvent are metered in with stirring at elevated temperature,
c) after complete reaction a phase transfer catalyst and a base are added,
d) a carboxylic acid derivative of formula (IV) is metered in, and
e) where the substituents are as defined hereinbelow: 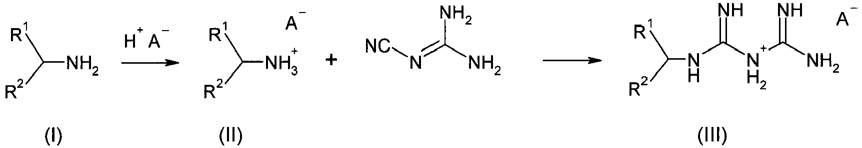 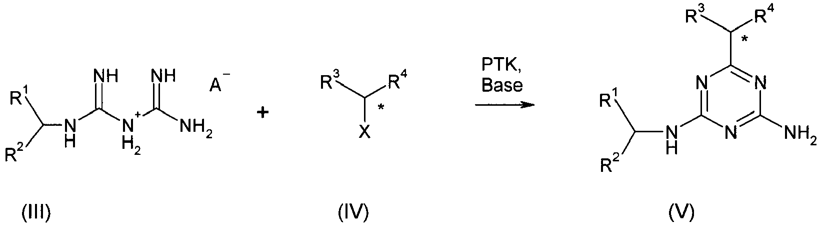
R¹ is hydrogen, (C₁-C₈) -alkyl, (C₃-C₈) -cycloalkyl, phenyl, naphthyl, (C₁-C₄)-alkylphenyl, wherein the four last-mentioned radicals are substituted by n radicals from the group consisting of methyl, ethyl, propyl and methoxy,
R² is hydrogen, (C₁-C₈)-alkyl, (C₃-C₈)-cycloalkyl, phenyl, naphthyl, phenyl-(C₁-C₄)-alkyl, wherein these four abovementioned radicals are substituted by n radicals from the group consisting of methyl, ethyl, propyl, methoxy and (C₃-C₄)-cycloalkyl,
or
R¹ and R² together with the carbon atom to which they are bonded form a radical from the group consisting of (C₃-C₈)-cycloalkyl, phenyl-(C₃-C₈)-cycloalkyl, 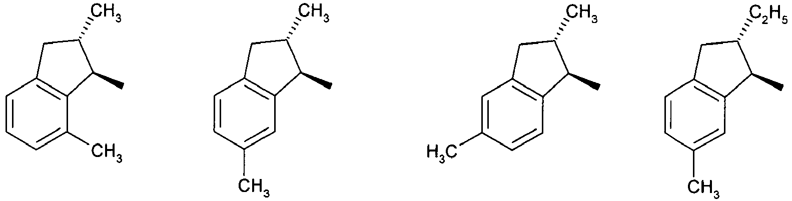 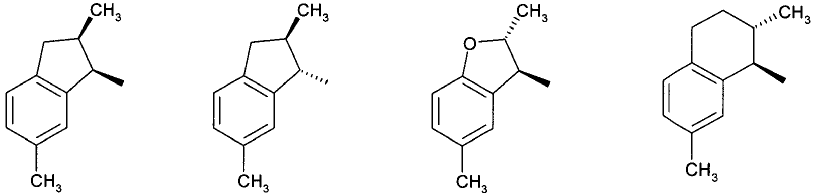 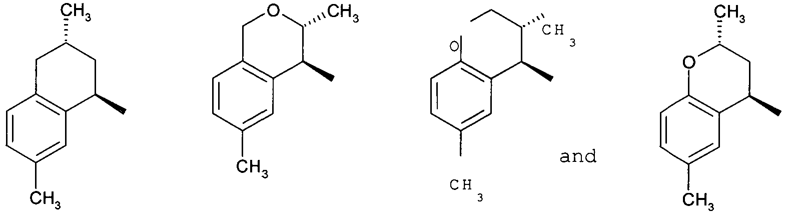
A⁻ is an anion of an acid,
n is 0, 1, 2 or 3,
R³ is hydrogen, halogen, (C₁-C₆)-alkyl, (C₁-C₆)-alkoxy, (C₃-C₈)-cycloalkyl, phenyl, wherein the three last-mentioned radicals are substituted by n radicals from the group consisting of methyl, ethyl, propyl and methoxy,
R⁴ is hydrogen, halogen, (C₁-C₆)-alkyl, (C₁-C₆)-alkoxy, (C₃-C₈)-cycloalkyl, phenyl, wherein the three last-mentioned radicals are substituted by n radicals from the group consisting of methyl, ethyl, propyl and methoxy,
X is (C₁-C₆)-alkoxycarbonyl, cyano or chlorocarbonyl.

5. Process according to Claim 4, wherein
R¹ is hydrogen, (C₁-C₈)-alkyl, (C₃-C₈)-cycloalkyl, phenyl, (C₁-C₄)-alkylphenyl, wherein the three last-mentioned radicals are substituted by n radicals from the group consisting of methyl, ethyl, propyl and methoxy,
R² is (C₁-C₈)-alkyl, (C₃-C₈)-cycloalkyl, phenyl, phenyl-(C₁-C₄)-alkyl,
or
R¹ and R² together with the carbon atom to which they are bonded form a radical from the group consisting of (C₃-C₈)-cycloalkyl, phenyl-(C₃-C₈)-cycloalkyl, 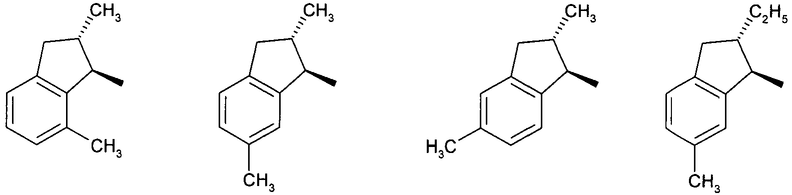 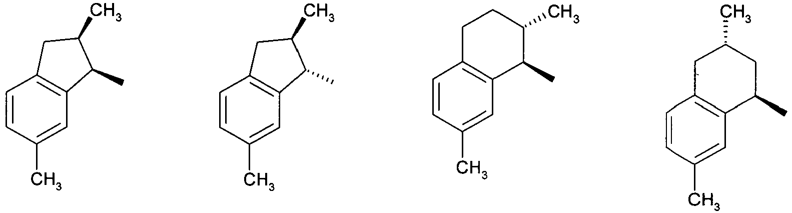 A⁻ is Cl⁻, Br⁻ or HSO₄⁻,
n is 0, 1, 2 or 3,
R³ is hydrogen, chlorine, fluorine, (C₁-C₆)-alkyl or (C₁-C₆)-alkoxy,
R⁴ is hydrogen, chlorine, fluorine, (C₁-C₆)-alkyl or (C₁-C₆)-alkoxy,
X is (C₁-C₆)-alkoxycarbonyl.

6. Process according to Claim 4 or 5, wherein
R¹ and R² together with the carbon atom to which they are bonded form the radical 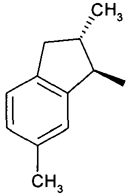 A⁻ is Cl⁻,
R³ is fluorine,
R⁴ is methyl,
X is (C₁-C₆)-alkoxycarbonyl.

7. Process according to any of Claims 1 to 6, wherein the reaction in step b) is carried out at a temperature from 100°C to the boiling point of the solvent and with an excess of 5% to 10% of cyanoguanidine based on the amine salt of formula (II).

8. Process according to Claim 7, wherein the reaction in step b) is carried out in anisole.

9. Process according to any of Claims 1 to 8, wherein the reaction in step b) is carried out with 0.5 to 2 mol percent of the biguanidine salt (III) to be produced.

10. Process according to any of Claims 4 to 6, wherein the reaction in step c) is carried out with 0.5 to 5 mol percent, based on the amine of formula (I), of a polyethylene glycol as the phase-transfer catalyst.

11. Process according to any of Claims 4 to 6, wherein the reaction in step c) is carried out with 100-400 mol percent, based on the amine of formula (I), of a base from the group consisting of potassium carbonate, sodium carbonate, lithium carbonate, calcium carbonate and magnesium carbonate.

12. Process according to Claim 11, wherein the reaction is carried out with 100-150 mol percent of potassium carbonate based on the amine of formula (I).

## Revendications

1. Procédé de fabrication de sels de biguanidine de formule (III), qui est **caractérisé en ce que**
a) une amine de formule (I) est transformée en un sel (II) avec un acide H⁺A⁻,
b) sous agitation à température élevée, de la cyanoguanidine et 0,5 à 10 pour cent en moles du sel de biguanidine (III) à fabriquer, par rapport à l'amine de formule (I), sont ajoutés dans un solvant aprotique polaire,
c) le mélange réactionnel ainsi obtenu est aspiré après refroidissement, lavé avec un solvant et séché, et
d) les substituants sont définis de la manière suivante : 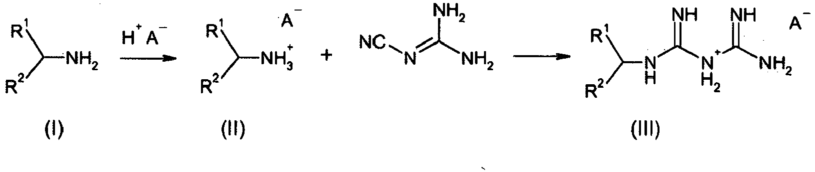
R¹ signifie hydrogène, alkyle en (C₁-C₈), cycloalkyle en (C₃-C₈), phényle, naphtyle, alkylphényle en (C₁-C₄), les quatre derniers radicaux mentionnés étant substitués par n radicaux du groupe constitué par méthyle, éthyle, propyle et méthoxy,
R² signifie alkyle en (C₁-C₈), cycloalkyle en (C₃-C₈), phényle, naphtyle, phényl-alkyle en (C₁-C₄), ces quatre radicaux susmentionnés étant substitués par n radicaux du groupe constitué par méthyle, éthyle, propyle, méthoxy et cycloalkyle en (C₃-C₄), ou
R¹ et R² forment ensemble avec l'atome de carbone auquel ils sont reliés un radical du groupe constitué par cycloalkyle en (C₃-C₈), phényl-cycloalkyle en (C₃-C₈), 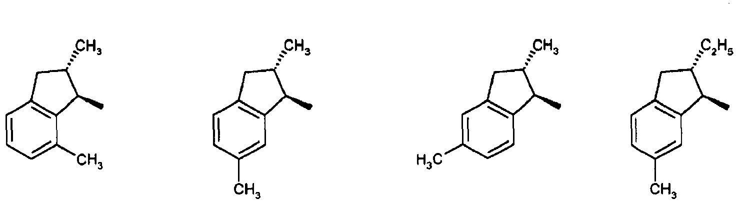 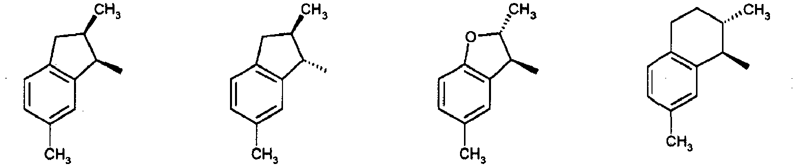 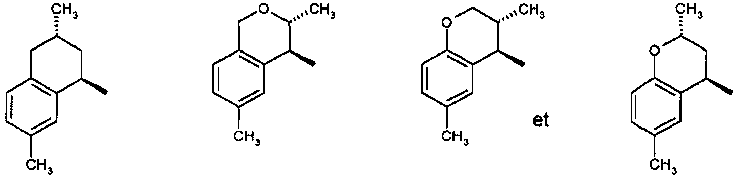
A⁻ signifie un anion d'un acide,
n signifie 0, 1, 2 ou 3.

2. Procédé selon la revendication 1, dans lequel R¹ signifie hydrogène, alkyle en (C₁-C₈), cycloalkyle en (C₃-C₈), phényle, alkylphényle en (C₁-C₄), les trois derniers radicaux mentionnés étant substitués par n radicaux du groupe constitué par méthyle, éthyle, propyle et méthoxy,
R² signifie alkyle en (C₁-C₈), cycloalkyle en (C₃-C₈), phényle, naphtyle ou phényl-alkyle en (C₁-C₄),
ou
R¹ et R² forment ensemble avec l'atome de carbone auquel ils sont reliés un radical du groupe constitué par cycloalkyle en (C₃-C₈), phényl-cycloalkyle en (C₃-C₈), 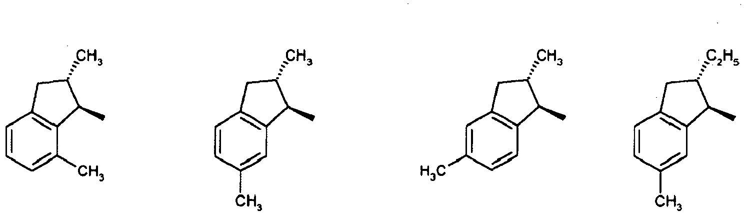 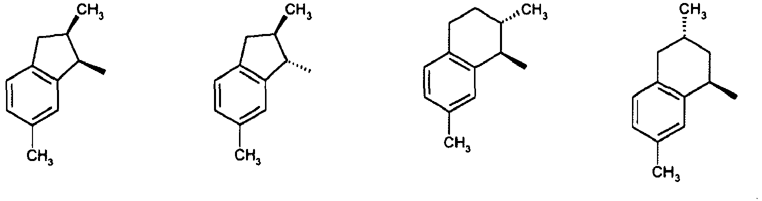 A⁻ signifie Cl⁻, Br⁻ ou HSO₄⁻,
n signifie 0, 1, 2 ou 3.

3. Procédé selon la revendication 1 ou 2, dans lequel R¹ et R² forment ensemble avec l'atome de carbone auquel ils sont reliés le radical 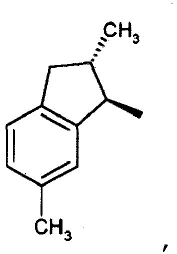 et
A⁻ signifie Cl⁻.

4. Procédé de fabrication de s-triazines de formule (V), qui est **caractérisé en ce que**
a) une amine de formule (I) est transformée en un sel (II) avec un acide H⁺A⁻,
b) sous agitation à température élevée, de la cyanoguanidine et 0,5 à 10 pour cent en moles du sel de biguanidine (III) à fabriquer, par rapport à l'amine de formule (I), sont ajoutés dans un solvant aprotique polaire,
c) après la réaction totale, un catalyseur de transfert de phases et une base sont ajoutés,
d) un dérivé d'acide carboxylique de formule (IV) est ajouté, et
e) les substituants sont définis de la manière suivante : 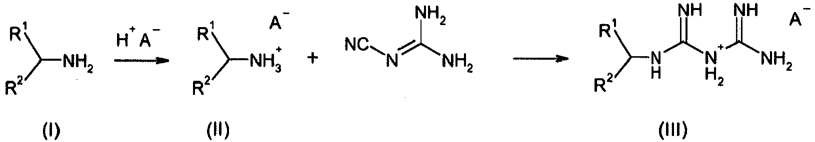 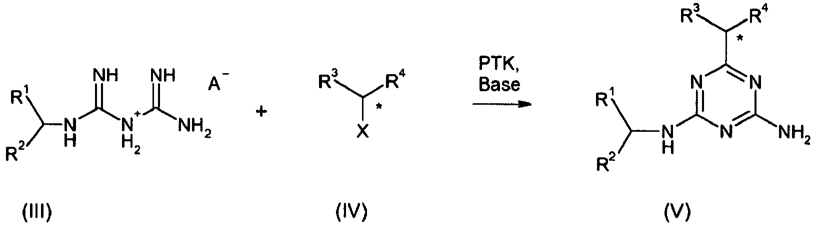
R¹ signifie hydrogène, alkyle en (C₁-C₈), cycloalkyle en (C₃-C₈), phényle, naphtyle, alkylphényle en (C₁-C₄), les quatre derniers radicaux mentionnés étant substitués par n radicaux du groupe constitué par méthyle, éthyle, propyle et méthoxy,
R² signifie alkyle en (C₁-C₈), cycloalkyle en (C₃-C₈), phényle, naphtyle, phényl-alkyle en (C₁-C₄), ces quatre radicaux susmentionnés étant substitués par n radicaux du groupe constitué par méthyle, éthyle, propyle, méthoxy et cycloalkyle en (C₃-C₄), ou
R¹ et R² forment ensemble avec l'atome de carbone auquel ils sont reliés un radical du groupe constitué par cycloalkyle en (C₃-C₈), phényl-cycloalkyle en (C₃-C₈), 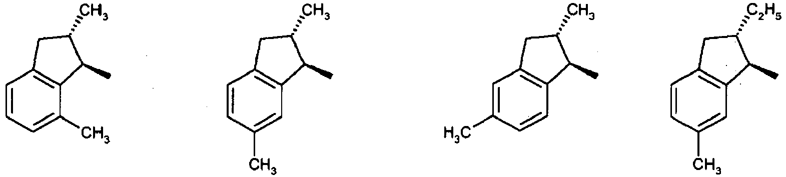 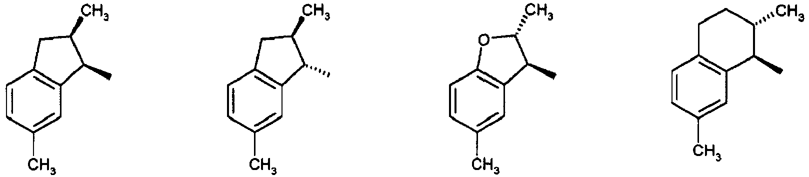 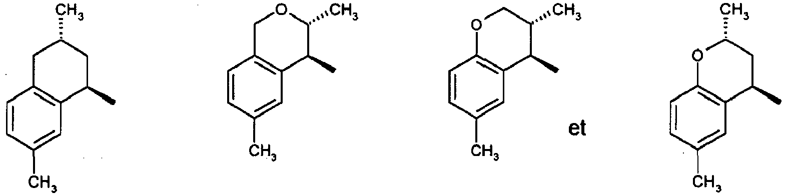
A⁻ signifie un anion d'un acide,
n signifie 0, 1, 2 ou 3,
R³ signifie hydrogène, halogène, alkyle en (C₁-C₆), alcoxy en (C₁-C₆), cycloalkyle en (C₃-C₈), phényle, les trois derniers radicaux mentionnés étant substitués par n radicaux du groupe constitué par méthyle, éthyle, propyle et méthoxy,
R⁴ signifie hydrogène, halogène, alkyle en (C₁-C₆), alcoxy en (C₁-C₆), cycloalkyle en (C₃-C₈), phényle, les trois derniers radicaux mentionnés étant substitués par n radicaux du groupe constitué par méthyle, éthyle, propyle et méthoxy,
X signifie alcoxycarbonyle en (C₁-C₆), cyano ou chlorocarbonyle.

5. Procédé selon la revendication 4, dans lequel R¹ signifie hydrogène, alkyle en (C₁-C₈), cycloalkyle en (C₃-C₈), phényle, alkylphényle en (C₁-C₄), les trois derniers radicaux mentionnés étant substitués par n radicaux du groupe constitué par méthyle, éthyle, propyle et méthoxy,
R² signifie alkyle en (C₁-C₈), cycloalkyle en (C₃-C₈), phényle, phényl-alkyle en (C₁-C₄), ou
R¹ et R² forment ensemble avec l'atome de carbone auquel ils sont reliés un radical du groupe constitué par cycloalkyle en (C₃-C₈), phényl-cycloalkyle en (C₃-C₈), 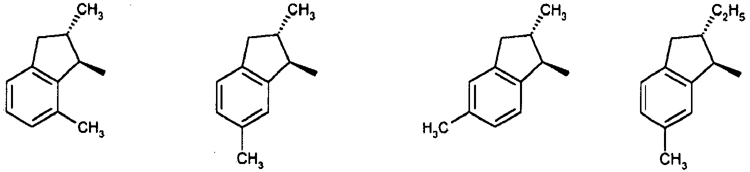 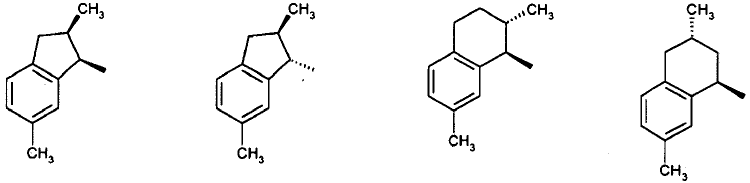 A⁻ signifie Cl⁻, Br⁻ ou HSO₄⁻,
n signifie 0, 1, 2 ou 3,
R³ signifie hydrogène, chlore, fluor, alkyle en (C₁-C₆) ou alcoxy en (C₁-C₆),
R⁴ signifie hydrogène, chlore, fluor, alkyle en (C₁-C₆) ou alcoxy en (C₁-C₆),
X signifie alcoxycarbonyle en (C₁-C₆).

6. Procédé selon la revendication 4 ou 5, dans lequel R¹ et R² forment ensemble avec l'atome de carbone auquel ils sont reliés le radical 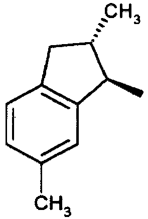 A⁻ signifie Cl⁻,
R³ signifie fluor,
R⁴ signifie méthyle,
X signifie alcoxycarbonyle en (C₁-C₆).

7. Procédé selon l'une quelconque des revendications 1 à 6, dans lequel la réaction à l'étape b) est réalisée à une température de 100 °C jusqu'au point d'ébullition du solvant et avec un excès de 5 à 10 % de cyanoguanidine, par rapport au sel d'amine de formule (II) .

8. Procédé selon la revendication 7, dans lequel la réaction à l'étape b) est réalisée dans de l'anisole.

9. Procédé selon l'une quelconque des revendications 1 à 8, dans lequel la réaction à l'étape b) est réalisée avec 0,5 à 2 pour cent en moles du sel de biguanidine (III) à fabriquer.

10. Procédé selon l'une quelconque des revendications 4 à 6, dans lequel la réaction à l'étape c) est réalisée avec 0,5 à 5 pour cent en moles, par rapport à l'amine de formule (I), d'un polyéthylène glycol en tant que catalyseur de transfert de phases.

11. Procédé selon l'une quelconque des revendications 4 à 6, dans lequel la réaction à l'étape c) est réalisée avec 100 à 400 pour cent en moles, par rapport à l'amine de formule (I), d'une base du groupe constitué par le carbonate de potassium, le carbonate de sodium, le carbonate de lithium, le carbonate de calcium et le carbonate de magnésium.

12. Procédé selon la revendication 11, dans lequel la réaction est réalisée avec 100 à 150 pour cent en moles de carbonate de potassium, par rapport à l'amine de formule (I).
